# EUROPÄISCHE PATENTSCHRIFT

(11) **EP 2 991 591 B1**
(45) Veröffentlichungstag und Bekanntmachung des Hinweises auf die Patenterteilung: **31.01.2018**
(21) Anmeldenummer: 14723326.6
(22) Anmeldetag: 29.04.2014
(51) Int. Cl.: A61F 2/58, A61F 2/78

(54) **KÜNSTLICHER FINGER**
ARTIFICIAL FINGER
DOIGT ARTIFICIEL

(30) Priorität: 03.05.2013 DE 102013007539
(43) Veröffentlichungstag der Anmeldung: 09.03.2016
(60) Teilanmeldung: 17210350.9
(73) Patentinhaber: Otto Bock Healthcare Products GmbH, 1070 Wien (AT)
(72) Erfinder: MANDL, Clemens, 1030 Wien (AT); OECKER, Leopold, 1220 Wien (AT); SKIERA, Richard, 1210 Wien (AT); VAN VLIET, Johannis, Willem, 3193 St. Aegyd Am Neuwalde (AT); NADERER, Ronald, 4181 Oberneukirchen (AT); FERRARA, Paolo, 4531 Kematen an der Krems (AT); SCHAUSBERGER, Florian, 4040 Linz (AT)
(74) Vertreter: Stornebel, Kai
(86) Internationale Anmeldenummer: PCT/EP2014/001143
(87) Internationale Veröffentlichungsnummer: WO 2014/177272

(56) Entgegenhaltungen:
- EP-A1- 0 045 818
- WO-A1-2010/051798
- US-A- 5 108 443
- US-A1- 2007 213 831

## Beschreibung

Die Erfindung betrifft einen künstlichen Finger mit einer Basis, einem an der Basis gelenkig gelagerten ersten Glied und zumindest einem gelenkig an dem ersten Fingerglied gelagerten Folgeglied sowie einem Antrieb zum Verstellen des Folgegliedes relativ zu dem ersten Fingerglied und des ersten Fingergliedes zu der Basis.

Künstliche Finger werden in der Prothetik ebenso wie in der Handhabungstechnik, bei der Roboter oder Handhabungsautomaten mit fingerbehafteten Greifern ausgestattet sind, eingesetzt. Bei einem Einsatz an Prothesenhänden werden Prothesenfinger benötigt, um nicht vorhandene Finger einer natürlichen Hand funktional oder optisch zu ersetzen. Bei Robotern oder Handhabungsautomaten sollen die künstlichen Finger die Flexibilität der menschlichen Hand nachbilden. Nachfolgend sind sowohl technische Greifelemente als auch prothetische Komponenten gemeint, wenn allgemein von Fingern gesprochen wird, anderenfalls wird gesondert auf die Art der Finger hingewiesen. Unter Greifeinrichtungen sind sowohl Prothesenhände als auch technische Greifer, Endeffektoren und Handhabungseinrichtungen zu verstehen. Neben passiven Prothesenfingern, die überwiegend eine kosmetische Funktion aufweisen und nicht aktuierbar an einer Basis befestigt sind, ist es zum Bereitstellen einer Greifeinrichtung, mit der Gegenstände gegriffen werden können, notwendig, Finger relativ zu einer Basis zu aktuieren. Dazu ist im einfachsten Fall ein starrer Finger gelenkig an der Basis gelagert und mit einem Seilzugsystem gekoppelt, das gegen eine Rückstellfeder bei einer Aktuierung eine Flexion bewirkt.

Aus der WO 2010/018358 A2 sind eine Vielzahl unterschiedlicher Aktuierungsmechanismen für Prothesenfinger bekannt. Neben Seilzügen, die direkt über an der Basis befestigte Motoren betätigbar oder an einem verlagerbaren Joch befestigt sind, sind Direktantriebe durch Zahnräder oder Spindeln zur Verlagerung der Proximalglieder relativ zu der Basis offenbart. Darüber hinaus ist eine Kopplung eines Folgegliedes an einem Proximalglied über ein separates Bandelement gezeigt, so dass bei einer Flexion des Proximalgliedes eine automatische Flexion des Folgegliedes vorgenommen wird.
Die DE 600 23 142 T2 betrifft einen beweglichen Finger für eine Prothese mit einer Basis, in dem ein Antrieb gelagert ist. Über einen Draht, der an einer Spindelmutter befestigt ist, kann eine Flexion eines Zwischenabschnittes erfolgen, eine Rückstellung wird über eine Zwischenabschnittsfeder bei Revision des Antriebs vorgenommen. Über ein Seilzugsystem können mehrere distale Glieder abgewinkelt werden.
Die WO 2011/087382 A1 betrifft eine modular aufgebaute Prothesenhand mit mechanisch unabhängigen Fingermodulen, bei denen ein elektrischer Motor mit einem Getriebe innerhalb eines proximalen Abschnittes des Fingers angeordnet ist. Über ein Mehrlenkersystem kann bei Verstellung eines Schneckengetriebes ein Distalelement relativ zu einem Proximalelement verschwenkt werden, über eine Kopplungsstange wird gleichzeitig eine Kraft übertragen, um das Proximalelement gegenüber der Basis zu verschwenken.

Die EP 0 045 818 A1 und die WO 2010/051798 A1 betreffen einen künstlichen Finger mit den Merkmalen gemäß dem Oberbegriff von Anspruch 1.
Die US 2007/0213831 A1 betrifft einen Prothesenfinger oder eine Prothesenzehe mit einer Basis, einem an der Basis gelenkig gelagerten ersten Finger- oder Zehenglied und zumindest einem gelenkig an dem ersten Finger- oder Zehenglied gelagerten Folgeglied angeordnet ist, wobei an der Basis Einrichtungen zur osseointegrierten Festlegung des künstlichen Fingers oder der künstlichen Zehe angeordnet sind. Über ein Kopplungselement kann bei Überlast die Basis von den übrigen Prothesenkomponenten getrennt werden.
Aufgabe der vorliegenden Erfindung ist es, einen künstlichen Finger bereitzustellen, der bei einem einfachen Aufbau eine verbesserte Anlage an ein zu greifendes Objekt ermöglicht und vielseitig einsetzbar ist.

Erfindungsgemäß wird diese Aufgabe durch einen künstlichen Finger mit den Merkmalen des Hauptanspruches sowie mit den Merkmalen des nebengeordneten Anspruchs gelöst. Vorteilhafte Ausgestaltungen und Weiterbildungen der Erfindung sind in den Unteransprüchen, der Beschreibung und den Figuren offenbart.

Ein künstlicher Finger als Prothesenfinger mit einer Basis, einem an der Basis gelenkig gelagerten ersten Fingerglied und zumindest einem gelenkig an dem ersten Fingerglied gelagerten Folgeglied sowie einem motorischen Antrieb zum Verstellen des Folgegliedes relativ zu dem ersten Fingerglied und des ersten Fingergliedes zu der Basis sieht vor, dass an der Basis Einrichtungen zur osseointegrierten Festlegung des Prothesenfingers angeordnet sind und eine Steuereinrichtung für den Antrieb vorgesehen ist, die über elektromyographische Impulse oder direkte Ankoppelung an Nervenbahnen den Antrieb steuert, wodurch ein aktiver Prothesenfinger, der auf Grundlage elektromyografischer Impulse gesteuert werden kann, bereitgestellt werden kann. Auch ist es grundsätzlich möglich, eine unmittelbare Ankopplung einer Steuereinrichtung an Nervenbahnen vorzusehen, so dass ein nahezu vollständiger funktionaler Ersatz bei einem Verlust oder Nichtvorhandensein eines natürlichen Fingers gewährleistet werden kann. Die technische Ausstattung eines solchen Prothesenfingers kann der entsprechen, wie sie nachfolgend im Zusammenhang mit künstlichen Fingern beschrieben ist, wobei sämtliche Vorteile und Varianten der nachfolgend beschriebenen Ausgestaltung des Prothesenfingers auch bei einer osseointegrierten Variante verwirklicht werden können. Alle nachfolgend beschriebenen Ausgestaltungsformen und Varianten beziehen sich somit ausdrücklich auch auf osseointegrierte Prothesenfinger.

Der erfindungsgemäße künstliche Finger mit einer Basis, einem an der Basis gelenkig gelagerten Proximalglied und zumindest einem gelenkig an dem ersten Fingerglied gelagerten Folgeglied sowie einem Antrieb, insbesondere motorischen Antrieb, zum Verstellen des Folgegliedes relativ zu dem ersten Fingerglied und des ersten Fingergliedes zu der Basis sieht vor, dass zumindest ein Rückstellelement für die Rückstellung des ersten Fingergliedes und des Folgegliedes vorgesehen ist und das erste Fingerglied mit einer von dem Folgeglied abweichenden Rückstellkraft beaufschlagt ist. Durch die Beaufschlagung der Fingerlieder mit unterschiedlich großen Rückstellkräften ist es möglich, dass ein abgestimmtes Beuge- und Rückstellverhalten des künstlichen Fingers zu erreichen, was insbesondere bei unteraktuierten Fingern vorteilhaft ist, da das gewünschte Verhalten mit einem geringen Aufwand und nur einem Antrieb erreicht werden kann. Es ist nicht notwendig, dass mehrere Antriebe vorgesehen sind, um eine Anlege- oder Ablöseverhalte der Finger an den zu greifenden Gegenständen zu erzeugen.

Eine Variante der Erfindung sieht vor, dass das Rückstellelement mit einer Kraftübersetzungseinrichtung gekoppelt oder als Federelement mit über die Länge des Federelementes unterschiedlichen Federkonstanten ausgebildet ist. Die Kraftübersetzungseinrichtung kann als Hebel, Hebelsystem, Getriebe, Seilzugkonstruktion mit Rollenübersetzung Zahnradgetriebe, Reibradgetriebe oder dergleichen ausgebildet sein. Alternativ zu der Ausstattung mit einer Kraftübersetzungseinrichtung, die die Greif- und ggf. Rückstellkraft unterschiedlich auf die einzelnen Fingerglieder aufteilt, kann das Rückstellelement oder der Kraftspeicher, der dem Antrieb entgegenwirkt, als ein Federelement ausgebildet sein, dass über seine Länge unterschiedliche Federkonstanten aufweist, so dass an unterschiedlichen Stellen des Federelementes unterschiedlich große Rückstellkräfte wirken, die des Antriebskraft entgegenstehen, um ein gestuftes Anlegen oder Ablösen einzelner Fingerglieder zu ermöglichen. Das Federelement kann beispielsweise als Biegestab aus einem Metall oder Kunststoff mit über seiner Länge unterschiedlichen Querschnitten ausgebildet sein, um so ortsaufgelöst unterschiedliche Rückstellkräfte zu erzeugen.

Eine Variante der Erfindung sieht vor, dass eine erste Rückstellfeder das Folgeglied gegenüber dem ersten Fingerglied und eine zweite Rückstellfeder das erste Fingerglied gegenüber der Basis in Richtung auf eine Ausgangsstellung mit Rückstellkräften versieht, wobei die Federkonstanten der Rückstellfedern verschieden sind. Auf diese Art und Weise ist es möglich, eine Differenzialkinematik mit einem geringen Aufwand zu erzeugen, bei dem mit einem einzelnen Antrieb zwei Freiheitsgrade realisiert werden, die unabhängig von der Geometrie des zu greifenden Objektes eine möglichst hohe Griffsicherheit gewährleisten und gleichzeitig bei einem Einsatz als Prothesenfinger zu einem physiologischen Griffbild führen.

Eine Weiterbildung der Erfindung sieht vor, dass die Federkonstante der ersten Rückstellfeder größer als die Federkonstante der zweiten Rückstellfeder ist, so dass eine Verschwenkung des Folgegliedes relativ zu dem ersten Fingerglied einen größeren Kraftaufwand erfordert als eine Verschwenkung des ersten Fingergliedes relativ zu der Basis. Dadurch wird erreicht, dass der Finger zunächst um das Grundgelenk verschwenkt, also um die Schwenkachse zwischen dem ersten Fingerglied und der Basis und nur dann, wenn das erste Fingerglied an einem Objekt oder an einem Endanschlag anliegt, beginnt die Verlagerung des Folgegliedes relativ zu dem ersten Fingerglied, also eine Flexion um die Schwenkachse zwischen dem ersten Fingerglied und dem Folgeglied. Dadurch wird es ermöglicht, dass durch eine einfache Kraftübertragungskinematik eine Differenzialwirkung um mindestens zwei Gelenkeinrichtungen erfolgt, so dass die mindestens zwei Fingerglieder sequentiell betätigbar sind.

An dem Antrieb kann ein Zugmittel, insbesondere ein Gurt, Band oder ein Seil oder ein hochflexibler Draht gekoppelt sein, das wiederum an der Basis festgelegt ist. Dadurch ist es möglich, über eine hochflexible Seilführung bei einer einfachen Kinematik mit nur einem Antrieb mehrere Gelenke in einer kraftgesteuerten und von der Griffsituation abhängigen Art und Weise zu betätigen.

Das Zugmittel kann dabei um die Schwenkachse des Folgegliedes herum geführt sein, um an zumindest einem Kraftangriffspunkt jenseits der Schwenkachse des Folgegliedes angelegt zu werden, was eine stabile Zugmittelführung bei gleichzeitig verringertem Reibungsaufwand mit sich bringt. Es ist auch vorgesehen, dass das Zugmittel unterhalb der distalen Schwenkachse geführt ist, um eine einfache und unmittelbare Aufbringung der Zugkraft zur Verschwenkung des Folgegliedes zu bewirken. Vorteilhafterweise ist das Zugmittel an dem Folgeglied um zumindest eine Umlenkeinrichtung geführt, beispielsweise eine Umlenkrolle oder einen Bolzen mit einem niedrigen Reibbeiwert, so dass die Antriebsleistung nicht oder nur möglichst gering in Reibleistung umgewandelt wird.

Die Umlenkeinrichtung kann verschieblich oder verschwenkbar an dem Folgeglied gelagert sein, beispielsweise an einem Schwenkarm oder in einer Langloch- oder Kulissenführung, über die es möglich ist, über einen vorgegeben Weg einen Spielausgleich zu erreichen. Sofern das Zugmittel und insbesondere die Umlenkeinrichtung in Distalrichtung mit einer Vorspannkraft beaufschlagt ist, ergibt sich eine Grundvorspannung des Zugmittels zwischen dem Antrieb und dem Festlegungspunkt an der Basis, so dass ein Überlastschutz vorhanden ist, wenn z. B. nach Aufbringung einer Stoßlast das Folgeglied unbeabsichtigt flektiert und extendiert wird. Durch die Vorspannkraft ist der Finger unmittelbar wieder einsetzbar. Bei einer ungewollten Flexion, beispielsweise aufgrund einer Stoßbelastung, werden über das Zugmittel keine Kräfte auf den Antrieb übertragen, wodurch ein Überlastungsschutz durch eine Kraftentkopplung erreicht wird. Die Extension über die Nulllage hinaus, die sogenannte Hyperextension, kann auch vorteilhaft zum Greifen flacher Gegenstände eingesetzt werden.

Der Antrieb kann als selbsthemmender Linearantrieb ausgebildet sein, so dass nach Erreichen einer gewünschten Stellung, was beispielsweise über einen Sensor detektiert werden kann, kein weiterer Energieverbrauch notwendig ist, um die eingestellte Position des Folgegliedes und/oder des ersten Fingergliedes beizubehalten. Grundsätzlich ist es auch möglich und vorgesehen, dass der Antrieb nicht als Elektromotor mit einer Selbsthemmung ausgebildet ist, sondern beispielsweise als pneumatischer Aktor, der nicht selbsthemmend ist. Bei einer solchen Ausgestaltung gibt es bei vorhandenem Druck auch bei gesperrten Ventilen eine Krafteinwirkung, jedoch keinen Luftverbrauch. Zur Aufrechterhaltung der Griffkraft sind pneumatische Aktoren besonders geeignet. Antriebe ohne Selbsthemmung bieten auch den Vorteil eines Überlastschutzes. Gegenkräfte werden nur bis zu einer gewissen Grenze aufgenommen, darüber hinaus geben die Antriebe nach, wodurch der Antriebsstrang nicht über diese Grenze hinaus belastet werden kann. Sofern ein selbsthemmender Antrieb vorgesehen ist, ist ein Überlastmechanismus vorteilhaft. Zusätzlich erfüllen Antriebe ohne Selbsthemmung im Fall einer aktiven Kraftregelung auch die Funktion einer Spanners, weil der Antrieb immer mit der eingestellten Kraft am Zugmittel oder Seil zieht, so dass dieses nicht erschlaffen kann.

Der Antrieb kann ein Untersetzungsgetriebe aufweisen, das mit einer Rolle oder einem Spindelgetriebe gekoppelt ist, an der oder dem wiederum das Zugmittel befestigt ist, so dass kleine, schnell laufende Motoren, insbesondere Außenläufermotoren eingesetzt werden können, die bei einer entsprechenden Untersetzung ein präzises und dennoch kraftvolles Verlagern der Folge- und ersten Fingerglieder ermöglichen. Der Antrieb kann das Zugmittel auf eine Rolle aufrollen, beispielsweise eine vorgespannte Rolle, oder aber über ein Spindelgetriebe an der Spindelmutter festlegen, so dass bei einer Umkehrung der Drehrichtung das Zugmittel aktiv freigegeben wird.

Der Antrieb kann sowohl in dem ersten Fingerglied als auch in dem Folgeglied angeordnet sein, grundsätzlich ist es auch möglich, dass der Antrieb innerhalb eines Basiselementes, beispielsweise im Mittelhandbereich einer Prothesenhand, angeordnet ist. Bei Handhabungsgeräten entspricht diese Ausgestaltung einer Trennung des Greifermoduls in eine Antriebskomponente und eine Greiferkinematik. Als Greiferkinematik wird der Teil des Greifermoduls bezeichnet, der die Bewegung eines Aktuators in eine Bewegung der Finger oder der Wirkelemente umsetzt. Es ist auch möglich, dass mehrere Antriebe in den Fingerelementen angeordnet sind, um eine mehrstufige, individuelle Verlagerung zu ermöglichen. So ist es möglich, einen Finger herzustellen, der neben einer Lagerung eines ersten Fingergliedes oder Proximalgliedes an einer Basis, z.B. Mittelhandbasis, ein Medialglied und ein Folgeglied aufweist, wobei sowohl in dem ersten Fingerglied als auch in dem Medialglied ein Antrieb angeordnet ist. Der Medialantrieb ist dann das erste Fingerglied für das Folgeglied. Auch ist es möglich, bei einer Ausgestaltung mit zwei Fingergelenken den Zugmitteltrieb über ein erstes Folgeglied, das dann ein Medialglied ist, hinaus zum Folgeglied weiterzuleiten und eine abgestufte Ausgestaltung der Federraten für die Rückstellfedern in die Ausgangsstellung bereitzustellen. Dabei kann es vorgesehen sein, dass die Rückstellfeder für das erste Fingerglied relativ zu der Basis eine geringere Federrate als die Rückstellfeder für das Medialglied zum ersten Fingerglied aufweist, die wiederum geringer als die Federrate der Rückstellfeder zwischen dem Medialglied und dem Folgeglied ist. Auch bei einer Ausgestaltung mit zwei Folgegliedern ist es möglich und vorgesehen, dass nur ein einziger Antrieb vorhanden ist, der in dem ersten Fingerglied oder an der Basis gelagert ist, um bei einer entsprechenden Betätigung eine Flexion der Glieder zu bewirken. Es liegt in allen Fällen ein unteraktuiertes System mit weniger Aktuatoren als Freiheitsgraden vor.

Das erste Fingerglied und/oder das Folgeglied können hyperextendierbar gelagert sein, so dass es möglich ist, dass das jeweilige Fingerglied über die gestreckte Normalposition, in der in der Regel die Längsachsen der Fingerglieder fluchten, hinaus extendiert werden. Darüber hinaus ist es vorgesehen, dass der Umschaltpunkt des Kraftangriffs zwischen Flexion und Extension in dem extendierbaren oder auch hyperextendierbaren Fingerglied und/oder Folgeglied angeordnet ist, wobei der Krafteinleitungspunkt für die Verlagerung des Fingergliedes verlagerbar ausgebildet ist, so dass bei einer entsprechenden Verlagerung der Krafteinleitung ein Umschalten von Extension zu Flexion erfolgen kann, so dass zwei Bewegungen durch den künstlichen Finger ausgeführt werden können, je nach Positionierung des Kraftangriffs, ohne die Kraftrichtung in dem Zugmittel zu ändern. Der Krafteinleitungspunkt kann auf unterschiedliche Arten verändert werden, beispielsweise kraftgesteuert durch elastisch gelagerte Umlenkelemente, die durch die Kraft innerhalb des Zugmittels verlagert werden. Es ist auch möglich, Schalteinrichtungen wie Hebel, Schieber oder dergleichen vorzusehen, um durch Verlagerung wie Verdrehung oder Verschiebung von Umlenkelementen oder Führungen des Zugmittels den Krafteinleitungspunkt diesseits oder jenseits des Umschaltpunktes zu positionieren und eine dementsprechend unterschiedliche Verlagerungsrichtung des jeweiligen Fingergliedes zu erreichen. Die Verlagerung der Umlenkelemente oder Führungen kann auch motorisch erfolgen. Grundsätzlich ist es auch möglich und vorgesehen, dass die Verlagerung des Krafteinleitungspunktes diesseits und jenseits des Umschaltpunktes auch unabhängig von der Möglichkeit einer Hyperextension erfolgt, so dass eine Bewegungsumkehr der Fingerglieder aktiv auch bei unteraktuierten Systemen erfolgen kann.

An der Basis oder dem ersten Fingerglied können Einrichtungen zur osseointegrierten Festlegung des Prothesenfingers angeordnet sein, so dass aufgrund der autarken Ausgestaltung des Prothesenfingers bei einem integrierten Antrieb eine aktive, osseointegrierte Fingerprothese realisierbar ist.

Eine Alternative sieht vor, dass an der Basis oder dem ersten Fingerglied Einrichtungen zur Befestigung an Robotern oder Handhabungsgeräten angeordnet sind, so dass die Anwendung der künstlichen Finger neben der Prothetik auch in der Handhabungstechnik erfolgen kann. Die hierfür notwendigen Befestigungseinrichtungen unterscheiden sich von dem Einrichtungen zur osseointegrierten Festlegung, insbesondere können sie stabiler und größer ausgeführt sein, um größere Kräfte übertragen zu können und eine Biokompatibilität muss nicht gewährleistet sein.

Nachfolgend wird ein Ausführungsbeispiel der Erfindung anhand der beigefügten Figuren näher erläutert. Es zeigen:
- Figur 1 -: eine schematische Querschnittsansicht eines Prothesenfingers;
- Figur 2 -: eine Detaildarstellung eines Antriebs;
- Figur 3 -: Greifsituationen;
- Figur 3a -: eine Darstellung eines Prothesenfingers mit Kräften;
- Figur 4 -: eine Variante des künstlichen Fingers;
- Figur 5 -: eine Detailansicht der Überlastsicherung in Ausgangsstellung; sowie
- Figur 6 -: eine Überlastsicherung gemäß Figur 5 in Betätigungsstellung.

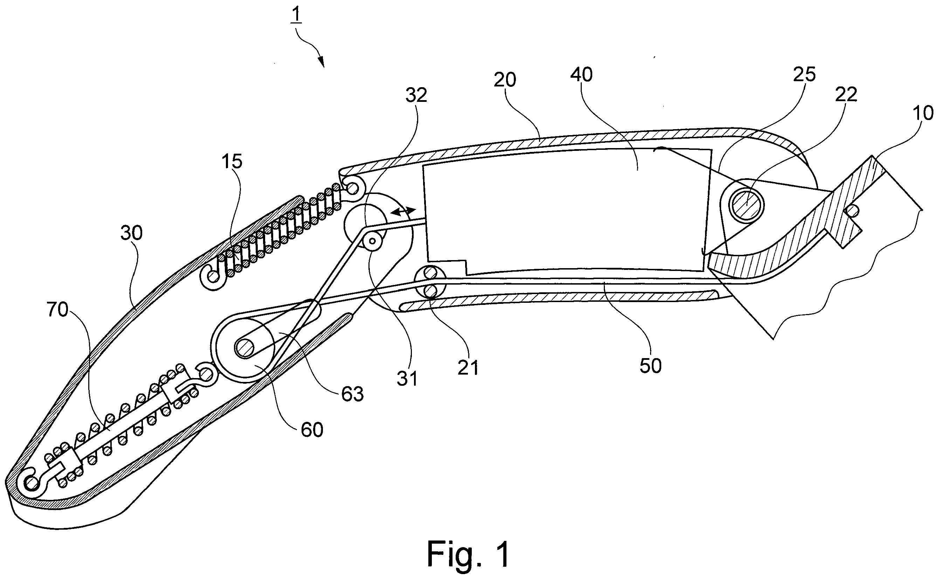

In der Figur 1 ist in einer schematischen Querschnittsansicht ein Finger 1 mit einer Basis 10 und einem um eine Gelenkachse 22 schwenkbar daran angeordneten Proximalglied 20 gezeigt. An dem distalen Ende des Ersten Fingergliedes 20 ist eine Schwenkachse 32 für ein darum schwenkbar gelagertes Folgeglied 30 vorgesehen.

Die Basis 10 kann als Metallplatte oder osseointegrierbare Komponente ausgebildet sein, die z.B. in einen Mittelhandknochen implantierbar ist.

Das Proximalglied 20 ist im dargestellten Ausführungsbeispiel hohl ausgebildet und weist in seinem Inneren einen motorischen Antrieb 40 auf, der später näher erläutert wird. An dem Antrieb 40 ist ein Zugmittel 50 befestigt, das aus einem distalen Ende des Antriebs 40 herausragt und in Längserstreckung des Ersten Fingergliedes 20 eine alternierende Bewegung ausführen kann, wie sie durch den Doppelpfeil dargestellt ist. Das Zugmittel 50, das als Gurt, Faserteil, Litze, Kunststofffaden, Sehne oder flexibler Draht ausgebildet sein kann, ist über eine Rolle 31 um die Schwenkachse 32 herum geführt und um ein Umlenkelement 60 geschlungen, das distal beabstandet zu der Schwenkachse 32 im Folgeglied 30 gelagert ist. Das Umlenkelement 60 ist in Gestalt einer Umlenkrolle ausgebildet, die in einer Langlochführung 63 verschieblich in dem Folgeglied 30 gelagert ist. Die Umlenkrolle 60 ist über eine Zugfeder 70 in Richtung auf das distale Ende des Folgegliedes 30 vorgespannt, so dass das Zugmittel 50 stets mit einer geringen Vorspannung behaftet ist. Dadurch ist es möglich, während der Benutzung auftretende Relativverlagerungen des Folgegliedes 30 zu dem ersten Fingerglied 20 auszugleichen, die nicht durch den Antrieb 40 oder Rückstellkräfte vorgesehen sind und eine Hyperextension zu ermöglichen.

Die Konstruktion des Fingers 1 mit dem Überlastschutz basiert darauf, dass die Zugfeder 70 derart vorgespannt ist, dass die Umlenkrolle 60 innerhalb der Langlochführung 63 erst ab einer gewissen Zugkraft, also ab einer bestimmten Zuglast im Zugmittel 50 in Richtung auf das erste Fingerglied 20 zu wandern beginnt, wobei die Federkraft so eingestellt ist, dass die Komponenten aufgrund einer Überlast nicht in Mitleidenschaft gezogen werden können. Der Finger 1 lässt sich somit auch öffnen, also das Folgeglied 30 bei einer Überlast extendieren. Die Zugfeder 70 ist daher relativ steif ausgelegt und mit einer Vorspannung versehen, die das Erreichen der maximalen Griffkraft ohne Vergrößerung der Federlänge aufnimmt.

Das Zugmittel 50 umschlingt die Umlenkrolle 60 und wird durch eine an dem ersten Fingerglied 20 befestigte Seilführung 21 in der Nähe der Außenwand des Ersten Fingergliedes 20 geführt, so dass der Verlauf der Zugmittels 50 im Wesentlichen parallel zur Gehäusewandung verläuft.

Zwischen dem ersten Fingerglied 20 und dem Folgeglied 30 ist eine erste Rückstellfeder 15 angeordnet, die einer Flexion des Folgegliedes 30 relativ zu dem ersten Fingerglied 20 aufgrund der Verkürzung der effektiven Zugmittellänge entgegenwirkt. Im dargestellten Ausführungsbeispiel ist die erste Rückstellfeder 15 als eine Zugfeder ausgebildet, die an einem Ende an dem ersten Fingerglied 20 und am anderen Ende an dem Folgeglied 30 festgelegt ist.

An dem ersten Fingerglied 20 ist als zweite Rückstellfeder 25 eine Schenkelfeder 25 um die Schwenkachse 32 herum angeordnet, die eine Rückstellkraft gegen eine Verschwenkung des Ersten Fingergliedes 20 relativ zu der Basis 10 bewirkt, so dass das erste Fingerglied bei einer maximalen Zugmittellänge in der gestreckten Stellung an einem Anschlag anliegt. Die Federrate der zweiten Rückstellfeder 25 ist dabei geringer als die Federrate der ersten Rückstellfeder 15, so dass bei einer Verkürzung der effektiven Zugmittellänge, also bei einem Hereinziehen des Zugmittels 50 in den Antrieb 40, zunächst um dasjenige Gelenk verschwenkt wird, das einen geringeren Widerstand entgegensetzt, im vorliegenden Ausführungsbeispiel also zunächst um das Gelenk zwischen der Basis 10 und dem ersten Fingerglied 20 um die Schwenkachse 32. Erst nach Erreichen eines Widerstandes, beispielsweise beim Anlegen des Ersten Fingergliedes 20 an einen Gegenstand, erhöht sich die Widerstandskraft bis auf einen Wert oberhalb der Rückstellkraft der ersten Rückstellfeder 15, was dann bei einer weiteren Verkürzung der effektiven Zugmittellänge zu einer Flexion des Folgegliedes 30 um die Schwenkachse 32 führt. Wird der Antrieb 40 in die entgegengesetzte Richtung betätigt, verlängert sich die effektive Zugmittellänge, beispielsweise indem das Zugmittel 50 von einer Rolle abgerollt oder eine Spindelmutter in Distalrichtung verlagert wird. Dann verlagert sich zunächst das Folgeglied 30 in eine Extensionsrichtung, erst nach Erreichen eines Extensionsanschlages für das Folgeglied 30 wird das erste Fingerglied 20 in Extensionsrichtung verlagert.

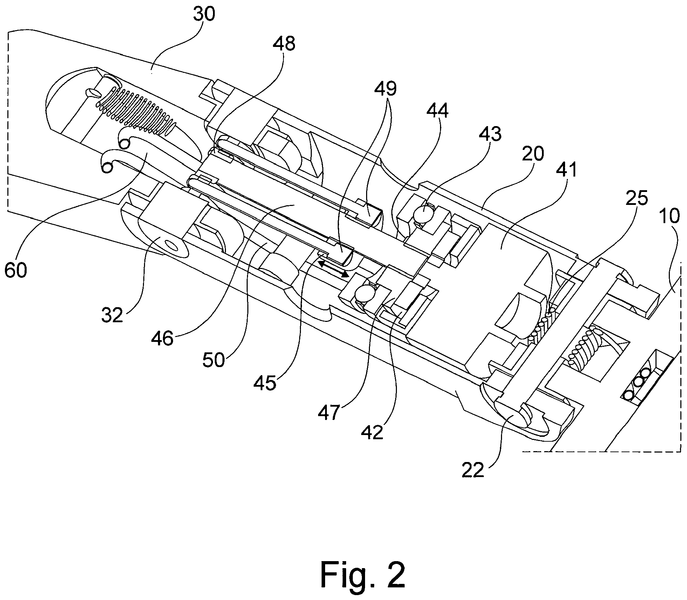

Figur 2 zeigt eine Detailansicht des Antriebs 40 mit einem Schnellläufermotor 41, der als Außenläufermotor ausgebildet ist. An den Außenläufermotor 41 schließt sich im eingebauten Zustand in Distalrichtung eine Planetengetriebestufe 42 an, dessen Planetenträger 47 Teil einer Bewegungsgewindespindel 46 ist. Der Planetenträger 47 stützt sich über ein Axialkugellager 43 auf einem nicht weiter dargestellten Gehäuse ab. Auf der Bewegungsgewindespindel 46 ist eine Spindelmutter 45 angeordnet, an der das Zugmittel 50 in Gestalt zweier Seile oder Drähte an beiderseits der Bewegungsgewindespindel positionierten Aufnahmen 49 befestigt ist. Am distalen Ende der Bewegungsgewindespindel 46 ist diese in einem Lager 48, beispielsweise ein Gleitlager oder Wälzlager, gelagert. An den jeweiligen Endbereichen der Bewegungsgewindespindel 46 sind Endlagenschalter 44 angeordnet, von denen nur der am distalen Anschlag dargestellt ist, über die der Außenläufermotor 41 abgeschaltet wird, wenn der maximale Verfahrweg der Spindelmutter 45 erreicht ist. Eine weitere Bewegung in diese Richtung ist dann nicht mehr möglich, erst nach Umkehrung der Bewegungsrichtung ist der Motor 41 wieder aktivierbar.

Durch Verfahren der Spindelmutter 45 in Proximalrichtung, also in Richtung auf den Motor 41, wird eine Zugkraft auf das Zugmittel 50 ausgeübt, wodurch durch Umlenkung an dem Umlenkelement 60 und abgestimmte Federkonstanten oder Steifigkeiten der Rückstellfedern 15, 25 eine sequentielle Schließung der Fingerglieder 20, 30 erreicht wird. Der Mechanismus ist sowohl mit zwei als auch mit drei Fingergliedern ausführbar, wobei die Ausgestaltung mit zwei Fingergliedern, wie sie in der Figur 1 dargestellt ist, den Vorteil einer natürlichen Fingerlänge aufweist. Mit drei Fingergliedern kann eine größere Annäherung an eine natürliche Fingerfunktion erreicht werden.

Der Antrieb 40 selbst kann sowohl in einem Fingerglied als auch in der Basis angeordnet sein, neben der Betätigung des Zugmittels 50 über das dargestellte Spindelgetriebe mit der Spindelmutter 45 ist es auch möglich, das Zugmittel 50 auf einer Rolle aufzurollen und wieder abzurollen. Neben der dargestellten Ausführungsform können als Antrieb auch Linearaktuatoren sowie pneumatische oder hydraulische Zylinder oder Bälge eingesetzt werden.

Durch die Erfindung ist es möglich, mit einer einfachen Kinematik mit nur einem Antrieb eine hohe Griffsicherheit durch eine Selbstanpassung der Finger an die Form der gegriffenen Gegenstände und bei Prothesenfingern ein physiologisches Griffbild beim Greifen verschiedener Objekte zu gewährleisten. Bei einem gegebenen Reibkoeffizienten des Gegenstandes und der Oberfläche der Fingerglieder hängt die Griffsicherheit hauptsächlich von der Griffkraft und dem Formschluss mit dem zu greifenden Objekt ab. Die vorliegende Erfindung erhöht den Formschluss unabhängig von der Geometrie des zu greifenden Objektes wesentlich, da ein sequentielles und vollständiges Anlegen beider oder aller Fingerglieder erreicht wird.

Ebenso bietet die erfindungsgemäße Ausgestaltung die Möglichkeit, den Finger passiv zu flektieren, was einem physiologischen Verhalten entspricht und die mechanischen Komponenten entlastet. Bei Handhabung in unstrukturierter Umgebung können Schäden, die durch Kollisionen entstehen, verringert werden. Die Möglichkeit zur passiven Flexion wird insbesondere durch die verlagerbare Umlenkrolle 60 gewährleistet. Aufgrund der Rückstellkraft über die Zugfeder 70 kann bei einer Stoßlast der Finger 1 unmittelbar wieder eingesetzt werden. Bei dem dargestellten Ausführungsbeispiel handelt es sich um ein sogenanntes unteraktuiertes System, bei dem die Anzahl der Aktuatoren geringer ist als die Anzahl der Freiheitsgrade. Bei kinematischer Kopplung der Gelenke über das Zugmittel kann auch bei einem feststehenden Antrieb ein Glied beugen, während andere gestreckt werden. Dadurch bleibt der Formschluss auch bei Veränderungen oder Verlagerungen des gegriffenen Objektes aufrecht.

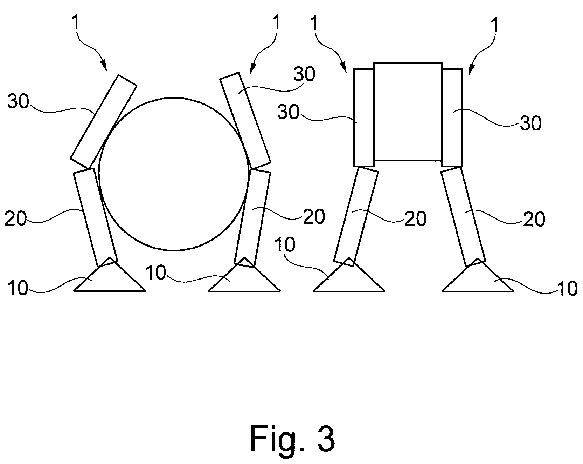

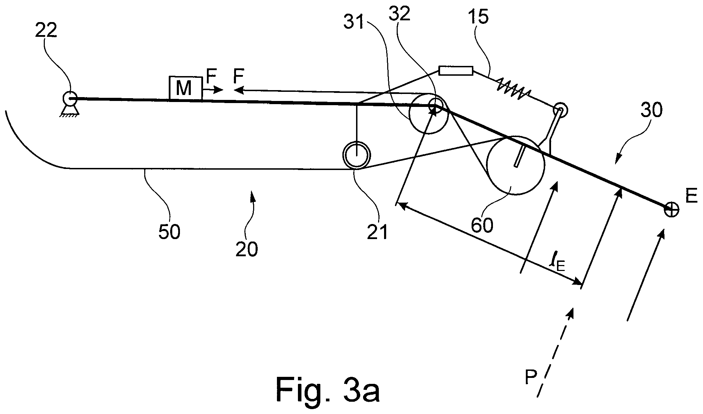

In der Figur 3 sind zwei verschiedene Greifsituationen dargestellt, wie sie für Finger gemäß der Figuren 1, 2, 3a und 4 auftreten können. Die linke Darstellung zeigt das Greifen eines Gegenstandes mit rundem Querschnitt, die rechte Darstellung zeigt das Greifen eines Gegenstandes mit einem eckigen, flachen Querschnitt.

In der linken Darstellung der Figur 3 ist gezeigt, dass sich nach einem Erstkontakt des ersten Fingergliedes 20, die gelenkig an der Basis 10 befestigt sind, das Folgeglied 30 um den Querschnitt des zu greifenden Gegenstandes herum anlegt, wenn die beiden Basen 10 der Finger 1 enger zusammenstehen als der maximale Durchmesser des zu greifenden Gegenstandes ist. Die ersten Fingerglieder 20 müssen dann zunächst aufgespreizt werden, so dass sie zunächst an der unteren Hälfte des zu greifenden Gegenstandes anliegen. Wird der nicht dargestellte Antrieb weiterhin betätigt, legen sich die Folgeglieder 30 um den Gegenstand und können diesen sicher halten.

Eine andere Greifsituation ist in der rechten Darstellung gezeigt, bei der die beiden Finger 1 mit ihren Basen 10 weiter auseinanderstehen als der zu greifende Gegenstand breit ist, also eine Greifsituation, in der ein schmaler oder flacher Gegenstand gegriffen werden soll. Die Extension des distalen Fingergliedes oder Folgegliedes 30 über 0° oder eine Ausgangsstellung hinaus, was als Hyperextension der Folgeglieder 30 bezeichnet wird, bietet den Vorteil einer Anpassung an die Objektform und dadurch zu einem größeren Kontaktbereich, wodurch sich ein sichererer Griff und in der Prothetik auch ein physiologischeres Griffbild erreichen lässt.

Wird ein Glied, insbesondere ein Folgeglied 30, über seine Nulllage hinaus gestreckt, wobei die Nulllage die Ruhelage oder die Ausgangslage im unbetätigten Zustand darstellt, spricht man von einer Hyperextension. Diese Hyperextension ist beim Greifen flacher Gegenstände, wie sie in der Figur 3 rechts dargestellt sind, von Vorteil, weil sich die Spitzen der Folgeglieder 30 an das zu greifende Objekt anpassen. Die einzelnen Fingerglieder 20, 30 sind durch das Zugmittel 50 gekoppelt, so dass auch bei einem gesperrten Antrieb 40 die Beugung oder Flexion eines Gliedes 20, 30 bei gleichzeitiger Streckung oder Extension anderer Glieder des künstlichen Fingers möglich ist. Durch die kinematische Kopplung der Gelenke zwischen den jeweiligen Gliedern 20, 30 können diese Effekte beispielsweise auch während des Greifens eintreten, wenn die Spitzen des Folgegliedes ein Objekt vor den Vorgängergliedern berührt. Dann kommt es zu einer Streckung oder Extension der Fingerspitze, während die übrigen Glieder weiter beugen oder flektieren.

Durch die Anordnung der Umlenkrollen 21, 60, 31 und der entsprechenden Führung des Zugmittels 50 ist es möglich, das Kräftegleichgewicht zwischen den Fingergliedern 20, 30 so zu beeinflussen, dass eine Kraft auf das letzte Glied zu einer Streckung führt, während dieselbe Kraft bei anderen Gliedern in Beugerichtung wirkt. Dann wird zum Beispiel bei einem Griff auf kleine, flache Gegenstände die Kontaktkraft, die in der Fingerspitze entsteht, in Streckmomente für das Glied der Fingerspitze umgewandelt, gleichzeitig wird diese Kontaktkraft in Beugemomente für die Vorgängerglieder, also diejenigen Fingerglieder, die sich von der Fingerspitze in Richtung auf die Basis 10 erstrecken, gewandelt.

Unabhängig von einer Längenänderung des Zugmittels 50 entsteht dabei eine Hyperextension des letzten Gliedes oder der letzten Glieder, wenn gleichzeitig das oder die Vorgängerglieder gebeugt werden.

Bei einer Verwendung von Federn zur Spannung des Zugmittels 50 können einzelne Glieder auch gestreckt werden, wenn andere blockieren, weil sich die Länge des Zugmittels 50 erhöht und dadurch der Weg für das jeweilige Gelenk freigegeben wird.

Die Hyperextension ist zwar beim Greifen flacher Gegenstände gewünscht, beim Umschlingen größerer oder runder Gegenstände, wie sie in der Figur 3 in der linken Darstellung gezeigt sind, ist es jedoch nachteilig. Da die Finger unteraktuiert sind, kann die Hyperextension nicht aktiv durch separate Antriebe beeinflusst werden. Daher ist vorgesehen, dass die kinematische Anordnung der Umlenkrollen 21, 31, 60 so erfolgt, dass ein Umschaltpunkt P für das Bewirken einer Hyperextension entsteht. Der Umschaltpunkt P ist diejenige Stelle, die sich beispielsweise in der Nähe einer Fingerspitze befindet, an der die Wirkung einer externen Kraft entweder zu einer Streckung oder zu einer Beugung führt, je nachdem, an welcher Position die äußere Kraft angreift. Greift die äußere Kraft am von der Basis entfernten Ende des Fingergliedes an, also in distale Richtung zur Fingerspitze, führt dies zu einer Streckung des Gelenks und einer Hyperextension. Dies ist zum Beispiel beim Greifen eines dünnen flachen Gegenstandes der Fall. Wandert die Kraft in Richtung auf die Basis oder in proximale Richtung des Fingergliedes, dann ist die Streckung hingegen gehemmt, eine Beugung des betreffenden Gliedes ist jedoch weiterhin möglich. Es ist somit möglich, Gegenstände auch mit dem letzten Folgeglied aktiv zu greifen und einen Gegenstand mit dem Finger zu umschließen.

Die Umlenkrollen können an den Gliedern verlagerbar und einstellbar befestigt sein, so dass der Umschaltpunkt in bestimmten Grenzen frei wählbar ist. Werden einzelne der Umlenkrollen beweglich aufgehängt, gegebenenfalls gegen eine Federkraft, kann der Umschaltpunkt auch während des Greifvorganges in Abhängigkeit von den Kräften im Zugmittel verändert werden.

Figur 3a zeigt eine Skizze eines künstlichen Fingers mit einem ersten Glied 20 und einem Folgeglied 30, dessen grundsätzlicher Aufbau dem der Figur 1 entspricht. Nicht alle Komponenten des Fingers der Figur 1 sind aufgrund der besseren Übersichtlichkeit dargestellt. In der Figur 3a ist der Umschaltpunkt P gezeigt, bei dem zwischen einer gehemmten und einer freien Hyperextension umgeschaltet wird. Ist der Kraftangriffspunkt einer externen Kontaktkraft F_{E}, von der Schwenkachse 32 gesehen jenseits des Umschaltpunktes P angeordnet, verlängert sich der Wirkhebel IE und die Streckung des Folgegliedes 30 ist nicht mehr gehemmt, was zu einer Hyperextension um die Schwenkachse 32 führt. Gleichzeitig entsteht im ersten Glied 20 im Basisgelenk um die Schwenkachse 22 weiterhin ein Drehmoment in Flexionsrichtung. Befindet sich der Kraftangriffspunkt diesseits des Umschaltpunktes P, also näher zur Schwenkachse 32, ist aufgrund der kinematischen Anordnung der Umlenkrollen eine Streckung bei Aufbringung einer Zugkraft auf das Zugmittel 50 gehemmt, so dass der Finger 1 weiterhin in der gebeugten, dargestellten Stellung bleibt. Eine Hyperextension des Folgegliedes 30 kann nicht erfolgen. Ist die Umlenkrolle 60 beweglich an dem Folgeglied 30 gelagert, kann der Umschaltpunkt P verändert werden, bei einer federbelasteten Lagerung der Umlenkrolle 60 kann eine Verlagerung des Umschaltpunktes in Abhängigkeit von den auftretenden Zugkräften innerhalb des Zugmittels 50 erfolgen.

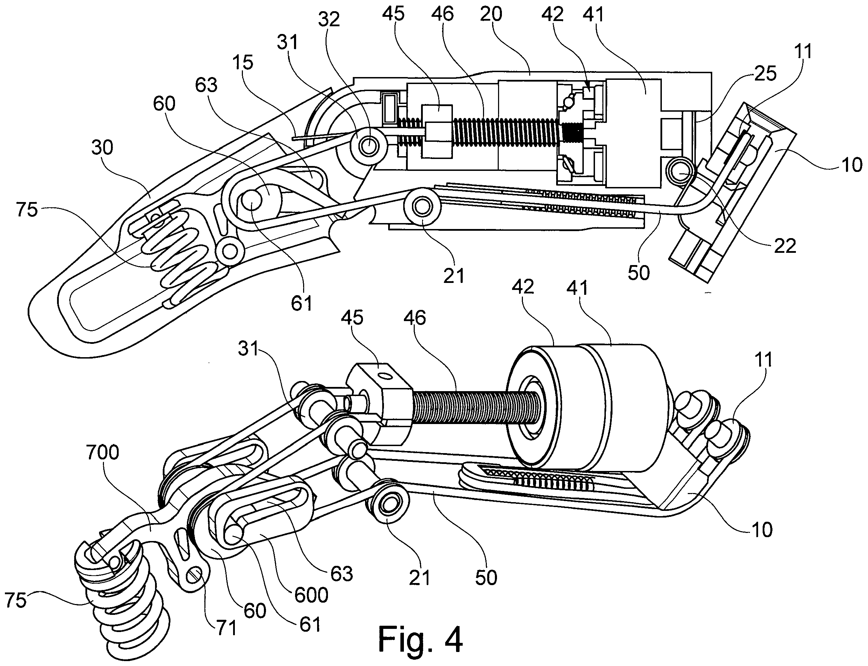

Die Figur 4 zeigt ein mechanisches Konzept eines künstlichen Fingers 1, das von dem der Figur 1 abweicht. Der grundsätzliche Aufbau mit einer Basis 10, einem um eine Schwenkachse 22 gelenkig daran gelagerten ersten Fingerglieds 20 und einem um die Schwenkachse 32 gelagerten Folgeglied 30 entsprechen der Ausführungsform gemäß Figur 1. Über den Antrieb 41, das Getriebe 42 und die Spindel 46 wird die Spindelmutter 45 entlang der Längserstreckung der Spindel 46 in Richtung auf den Antrieb 41 oder davon weg verlagert. Über das Zugmittel 50, das über mehrere Umlenkrollen 31, 60, 21, 11 geführt ist, wird eine Veränderung der effektiven Länge des Zugmittels 50 je nach Drehrichtung und Verlagerungsrichtung der Spindelmutter 45 bewirkt. Wird die Spindelmutter 45 in Richtung auf den Antrieb 41 verlagert, verkürzt sich die effektive Länge des Zugmittels 50 und das Folgeglied 30 wird flektiert. Die Federn 25, 15 mit den unterschiedlichen Federsteifigkeiten dienen zur Sicherstellung der sequentiellen Anlage der jeweiligen Fingerglieder 20, 30 an das zu greifende Objekt.

Die Umlenkrolle 60 ist um die Drehachse 61, um die herum beidseitig hervorstehende Zapfen ausgebildet sind, in einer Langlochführung 63 gelagert, die als Kulissenführung ausgebildet ist. Die Kulissenführung 63 ist in einem Rahmen 600 ausgebildet, zwei Rahmen sind beidseitig des parallel geführten Zugmittels 50 angeordnet und nehmen die Zapfen der Umlenkrolle 60 auf. Die Langlochführung 63 weist einen vorderen oder distalen Abschnitt auf, in dem die Zapfen der Umlenkrolle 60 in einer Ausgangsposition gelagert sind. Von diesem vorderen Bereich führt eine Schräge, die in einem Winkel von circa 70° zur Kraftrichtung K geführt ist, zu einem Gleitabschnitt, der im Wesentlichen in Kraftrichtung K orientiert ausgebildet ist. Über einen doppelarmigen Hebel 700, der um eine Schwenkachse 71, die parallel zu der Drehachse 61 der Umlenkrolle 600 orientiert ist, wird die Umlenkrolle 60 mit den Zapfen in dem vorderen Bereich gehalten. Der der Umlenkrolle 60 abgewandte Hebelarm stützt sich über eine Druckfeder 75 ab und presst die Zapfen der Umlenkrolle 60 in die Kulissenführung 63.

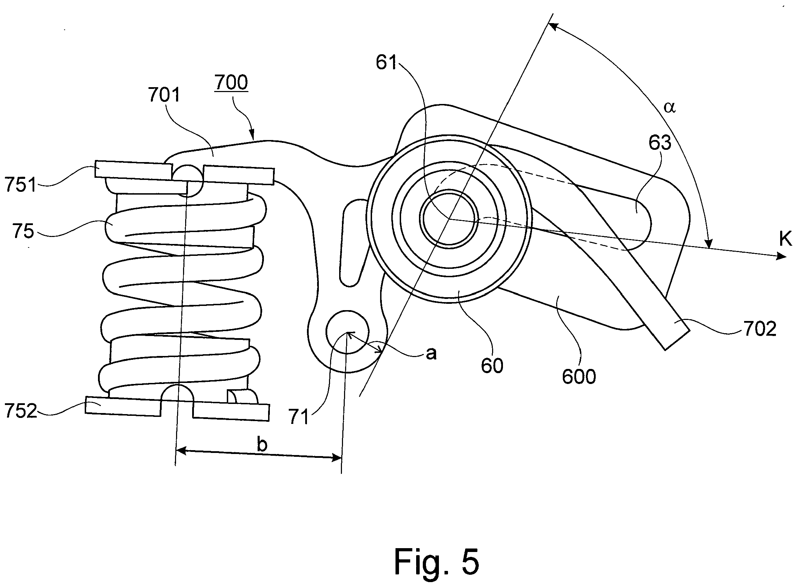

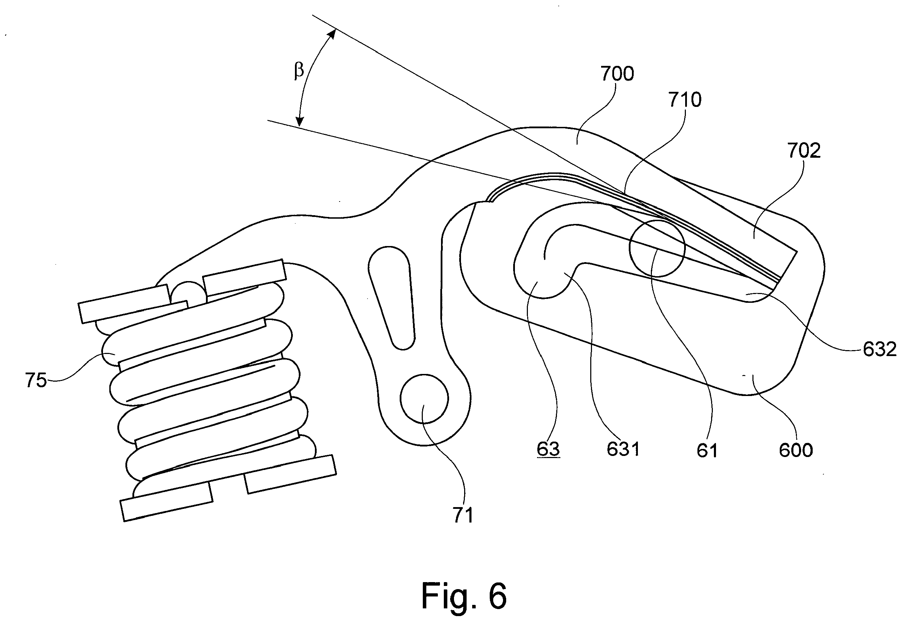

Die Funktionsweise dieser Überlastkonstruktion wird anhand der Figuren 5 und 6 näher erläutert. In der Figur 5 ist in einer vergrößerten Detaildarstellung die Anordnung gemäß Figur 4 in der Ausgangsposition gezeigt. Die Druckfeder 75 stützt sich über einen unteren Teller 752 an dem nicht dargestellten Folgeglied und über einen oberen Teller 751 an einem ersten Hebelarm 701 des Doppelhebels 700 ab. Über die Schwenkachse 71 wird über die Druckfeder 75 der Doppelhebel 700 in Uhrzeigerrichtung belastet, so dass die Umlenkrolle 60 über den zweiten Hebelarm 702, der eine gekrümmte, der Umlenkrolle 60 zugewandten Kontur aufweist, in den vorderen Abschnitt der Langlochführung 63 hineingedrückt wird. Der vordere Abschnitt lässt eine Verlagerung der Umlenkrolle 60 entlang einer Schräge zu, die einen Neigungswinkel α zur Kraftrichtung K aufweist, wobei die Kraftrichtung K durch die Führung des nicht dargestellten Zugmittels 50 und der Anordnung der entsprechenden Umlenkrollen 21, 31 festgelegt ist. Der Winkel α beträgt im dargestellten Ausführungsbeispiel ungefähr 70°, je nach Neigung der Schräge und Vergrößerung oder Verringerung des Neigungswinkels α werden größere Kräfte in Kraftrichtung K notwendig, um eine Kraftkomponente senkrecht zur Kraftrichtung K aufzubauen, so dass die Umlenkrolle 60 in dem vorderen Abschnitt entlanggleitet. Durch die Schräge, den Doppelhebel 700 und die gebogene Kontur des zweiten Hebelarms 702 ist eine Kraftverstärkung der Druckkraft der Druckfeder 75 gegeben, was dazu führt, dass eine relativ kurze Druckfeder 75 gewählt werden kann, um ausreichend hohe Haltekräfte für die Umlenkrolle 60 als Überlastsicherung bereitzustellen. Darüber hinaus muss der Federweg der Druckfeder 75 aufgrund der möglichen Hebelgeometrien nicht sehr groß sein, da aufgrund des Winkels α zwischen der Kraftrichtung K und der Orientierung der Schräge im ersten Abschnitt ein Großteil der Kraft durch die Reibung der Kulissenführung 63 aufkommen wird und nur ein geringer Kraftanteil durch die vorgespannte Druckfeder 75 aufgebracht werden muss.

Die Vorspannkraft der Druckfeder 75 wird auch durch die vorhandenen Hebelverhältnisse vergrößert. Das Verhältnis des ersten Krafthebels a zwischen der Schwenkachse 71 des Doppelhebels 700 und der Verlagerungsrichtung der Umlenkrolle entlang des vorderen Abschnittes der Kulissenführung zu dem zweiten Krafthebel b zwischen der Einleitstelle der Druckkraft in den ersten Hebelarm 701 und der Schwenkachse 71 des Doppelhebels beträgt im dargestellten Ausführungsbeispiel 1:4, so dass eine vierfache Kraftübersetzung auf die Drehachse 61 der Umlenkrolle ausgeübt werden kann. Die Schwenkachse 71 des Doppelhebels 700 liegt tiefer als die Drehachse 61 der Umlenkrolle 60 im vorderen Abschnitt der Kulissenführung 63, wodurch neben der Kraftübersetzung gewährleistet ist, dass nur wenig Federweg benötigt wird, um einen großen Verlagerungsweg in der Kulissenführung zu ermöglichen. Neben dem tiefliegenden Drehpunkt der Schwenkachse 71 weist der zweite Hebelarm 702 eine geneigte Kontur auf, die gewährleistet, dass in jedem Verlagerungspunkt der Drehzapfen der Umlenkrolle 60 ein definierter Reibwinkel β vorhanden ist.

Die Figur 6 zeigt den Reibwinkel β zwischen der unteren Kontur 710 des zweiten Hebelarms 702 im bereits verlagerten Zustand der nicht dargestellten Umlenkrolle 60 innerhalb der Kulissenführung 63. In dieser Darstellung ist auch der vordere Abschnitt 631 mit der Orientierung schräg zur Kraftrichtung K sowie der Gleitabschnitt 632 innerhalb des Rahmens 600 zu erkennen. Der angedeutete Lagerzapfen der Umlenkrolle 60 mit der Drehachse 61 befindet sich ungefähr in der Mitte des Gleitabschnittes 632, zwischen der Innenkontur 710 im Anlagepunkt des zweiten Hebels 702 und dem Verlauf der Kulissenführung 63 bildet sich aufgrund der gekrümmten Kontur 710 ein Reibwinkel β aus, der notwendig ist, um zu gewährleisten, dass nach Wegfall der Überlast sowohl die Umlenkrolle 60 als auch das Folgeglied 30 wieder in die Ausgangsstellung zurückbewegt wird. Hierzu ist der Reibwinkel β ausreichend groß gewählt, im dargestellten Ausführungsbeispiel 12°, was ausreichend ist, um bei Wegfall der Überlast durch den Druck von der komprimierten Druckfeder 75 über den ersten Hebelarm 701 um die Schwenkachse 71 eine Kraftkomponente entgegen der Kraftrichtung K und entlang des Bewegungsweges der Lagerzapfen der Umlenkrolle 60 innerhalb der Kulissenführung 63 zu bewirken. Der Reibwinkel β kann konstant über die Länge des zweiten Hebelarmes 702 sein, alternativ kann die Kontur 710 einen Zunehmenden oder abnehmenden Reibungswinkel β ausbilden, um ein gewünschtes Rückstellverhalten zu erreichen. Nach Wegfall der Überlast wird durch die Wahl des Reibwinkels β eine Kraftkomponente in Richtung auf den vorderen Abschnitt 631 ausgeübt, um die Rückstellung zu bewirken.

Die dargestellte Ausführungsform des Überlastschutzes gemäß den Figuren 4 bis 6 führt dazu, dass bei Erreichen einer Überlastkraft eine schlagartige Auslösung des Mechanismusses bewirkt, wodurch die durch den Finger 1 aufgebrachte Griffkraft schlagartig nachlässt. Sobald die aufgebrachte Flexionskraft nachlässt oder vollständig aufgehoben wird, wird die Umlenkrolle 60 wieder in die Ausgangsstellung gemäß Figur 5 zurückbewegt.

## Patentansprüche

1. Künstlicher Finger mit einer Basis (10), einem an der Basis (10) gelenkig gelagerten ersten Fingerglied (20) und zumindest einem gelenkig an dem ersten Fingerglied (20) gelagerten Folgeglied (30) sowie einem motorischen Antrieb (40) zum Verstellen des Folgegliedes (30) relativ zu dem ersten Fingerglied (20) und des Proximalgliedes (20) zu der Basis (10), **dadurch gekennzeichnet, dass** an der Basis (10) Einrichtungen zur osseointegrierten Festlegung des künstlichen Fingers (1) angeordnet sind und eine Steuereinrichtung für den Antrieb (40) vorgesehen ist, die über elektromyographische Impulse oder direkte Ankoppelung an Nervenbahnen den Antrieb (40) steuert.

2. Künstlicher Finger nach Anspruch 1, **dadurch gekennzeichnet, dass** zumindest ein Rückstellelement (15, 25) für die Rückstellung des ersten Fingergliedes (20) und des Folgegliedes (30) vorgesehen ist und das erste Fingerglied (20) mit einer von dem Folgeglied (30) abweichenden Rückstellkraft beaufschlagt ist.

3. Künstlicher Finger nach Anspruch 2, **dadurch gekennzeichnet, dass** das Rückstellelement (15, 25) mit einer Kraftübersetzungseinrichtung gekoppelt oder als Federelement mit über die Länge des Federelementes unterschiedlichen Federkonstanten ausgebildet ist.

4. Künstlicher Finger nach Anspruch 2, **dadurch gekennzeichnet, dass** eine erste Rückstellfeder (15) das Folgeglied (30) gegenüber dem ersten Fingerglied (20) und eine zweite Rückstellfeder (25) das erste Fingerglied (20) gegenüber der Basis (10) in Richtung auf eine Ausgangsstellung mit Rückstellkräften versieht, wobei die Federkonstanten der Rückstellfedern (15, 25) verschieden sind.

5. Künstlicher Finger nach Anspruch 4, **dadurch gekennzeichnet, dass** die Federkonstante der ersten Rückstellfeder (15) größer als die Federkonstante der zweiten Rückstellfeder (25) ist.

6. Künstlicher Finger nach einem der voranstehenden Ansprüche, **dadurch gekennzeichnet, dass** an dem Antrieb (40) ein Zugmittel (50) gekoppelt ist, das an der Basis (10) festgelegt ist.

7. Künstlicher Finger nach Anspruch 6, **dadurch gekennzeichnet, dass** das Zugmittel (50) unterhalb einer Schwenkachse (32) des Folgegliedes (30) geführt ist.

8. Künstlicher Finger nach einem der Ansprüche 6 oder 7, **dadurch gekennzeichnet, dass** das Zugmittel (50) an dem Folgeglied (30) um zumindest eine Umlenkeinrichtung (60) geführt ist.

9. Künstlicher Finger nach Anspruch 8, **dadurch gekennzeichnet, dass** die Umlenkeinrichtung (60) verschieblich oder verschwenkbar an dem Folgeglied (30) gelagert ist.

10. Künstlicher Finger nach einem der Ansprüche 6 bis 9, **dadurch gekennzeichnet, dass** das Zugmittel (50) in Distalrichtung mit einer Vorspannkraft beaufschlagt ist

11. Künstlicher Finger nach einem der voranstehenden Ansprüche, **dadurch gekennzeichnet, dass** der Antrieb (40) als selbsthemmender Linearantrieb oder nicht hemmender Pneumatikantrieb ausgebildet ist.

12. Künstlicher Finger nach einem der voranstehenden Ansprüche, **dadurch gekennzeichnet, dass** der Antrieb (40) ein Untersetzungsgetriebe (42) aufweist, das mit einer Rolle oder einem Spindelgetriebe (44) gekoppelt ist, an der oder dem das Zugmittel (50) befestigt ist.

13. Künstlicher Finger nach einem der voranstehenden Ansprüche, **dadurch gekennzeichnet, dass** der Antrieb (40) in dem ersten Fingerglied (20) oder Folgeglied (30) angeordnet ist.

14. Künstlicher Finger nach einem der voranstehenden Ansprüche, **dadurch gekennzeichnet, dass** ein Umschaltpunkt (P) eines Kraftangriffs zwischen Flexion und Extension in dem extendierbaren Fingerglied (20) und/oder Folgeglied (30) angeordnet und der Kraftangriffspunkt verlagerbar ausgebildet ist.

## Claims

1. Artificial finger with a base (10), a first finger member (20) mounted on the base (10) in an articulated manner, at least one secondary member (30) mounted on the first finger member (20) in an articulated manner, and a motor drive (40) for adjusting the secondary member (30) relative to the first finger member (20) and the proximal member (20) relative to the base (10), **characterized in that** devices for osseointegrated mounting of the artificial finger (1) are arranged on the base (10) and a control device is provided for the drive (40), which controls the drive (40) via electromyographical impulses or direct coupling to neural pathways.

2. Artificial finger according to claim 1, **characterized in that** at least one reset element (15, 25) is provided for resetting the first finger member (20) and the secondary member (30), and the first finger member (20) is acted upon with a resetting force which differs from the secondary member (30).

3. Artificial finger according to claim 2, **characterized in that** the reset element (15, 25) is coupled to a force transmission mechanism or is formed as a spring element with differing spring constants over the length of the spring element.

4. Artificial finger according to claim 2, **characterized in that** a first resetting spring (15) provides the secondary member (30) with resetting forces in relation to the first finger member (20) and a second resetting spring (25) provides the first finger member (20) with resetting forces in relation to the base (10) in the direction of a starting position, wherein the spring constants of the resetting springs (15, 25) are different.

5. Artificial finger according to claim 4, **characterized in that** the spring constant of the first resetting spring (15) is greater than the spring constant of the second resetting spring (25).

6. Artificial finger according to one of the preceding claims, **characterized in that** a traction means (50) is coupled to the drive (40) and fixed on the base (10).

7. Artificial finger according to claim 6, **characterized in that** the traction means (50) is guided below a pivot axis (32) of the secondary member (30).

8. Artificial finger according to claim 6 or 7, **characterized in that** the traction means (50) is guided on the secondary member (30) about at least one deflection device (60).

9. Artificial finger according to claim 8, **characterized in that** the deflection mechanism (60) is mounted displaceably or pivotably on the secondary member (30).

10. Artificial finger according to one of the claims 6 to 9, **characterized in that** a pre-tensioning force is applied to the traction means (50) in the distal direction.

11. Artificial finger according to one of the preceding claims, **characterized in that** the drive (40) is formed as a self-locking linear drive or a non-locking pneumatic drive.

12. Artificial finger according to one of the preceding claims, **characterized in that** the drive (40) has a reduction gear (42), which is coupled to a roller or a spindle drive (44), on which the traction means (50) is fixed.

13. Artificial finger according to one of the preceding claims, **characterized in that** the drive (40) is arranged in the first finger member (20) or the secondary member (30).

14. Artificial finger according to one of the preceding claims, **characterized in that** a switching point (P) of a force application between flexion and extension is arranged in the extendable finger member (20) and/or the secondary member (30), and the force application point is displaceable.

## Revendications

1. Doigt artificiel comportant une base (10), une première phalange (20) montée en articulation sur la base (10) et au moins une phalange successive (30) montée en articulation sur la première phalange (20) ainsi qu'un entraînement motorisé (40) pour déplacer la phalange successive (30) par rapport à la première phalange (20) et pour déplacer la phalange proximale (20) par rapport à la base (10),
**caractérisé en ce que** sur la base (10) sont agencés des moyens d'immobilisation ostéo-intégrée du doigt artificiel (1) et il est prévu un moyen de commande pour l'entraînement (40), qui commande l'entraînement (40) par des impulsions électro-myographiques ou par couplage direct à des voies neurales.

2. Doigt artificiel selon la revendication 1,
**caractérisé en ce que**
il est prévu au moins un élément de rappel (15, 25) pour rappeler la première phalange (20) et la phalange successive (30), et la première phalange (20) est sollicitée par une force de rappel qui diffère de celle de la phalange successive (30).

3. Doigt artificiel selon la revendication 2,
**caractérisé en ce que**
l'élément de rappel (15, 25) est couplé à un moyen multiplicateur de force ou est réalisé en tant qu'élément ressort ayant différentes constantes d'élasticité sur la longueur de l'élément ressort.

4. Doigt artificiel selon la revendication 2,
**caractérisé en ce que**
un premier ressort de rappel (15) sollicite la phalange successive (30) par des forces de rappel par rapport à la première phalange (20), et un second ressort de rappel (25) sollicite la première phalange (20) par des forces de rappel par rapport à la base (10) en direction d'une position de départ, les constantes d'élasticité des ressorts de rappel (15, 25) étant différentes.

5. Doigt artificiel selon la revendication 4,
**caractérisé en ce que**
la constante d'élasticité du premier ressort de rappel (15) est supérieure à la constante d'élasticité du second ressort de rappel (25).

6. Doigt artificiel selon l'une des revendications précédentes,
**caractérisé en ce que**
un moyen de traction (50) est couplé à l'entraînement, qui est immobilisé sur la base (10).

7. Doigt artificiel selon la revendication 6,
**caractérisé en ce que**
le moyen de traction (50) est guidé au-dessous d'un axe de basculement (32) de la phalange successive (30).

8. Doigt artificiel selon l'une des revendications 6 ou 7,
**caractérisé en ce que**
le moyen de traction (50) est guidé sur la phalange successive (30) autour d'au moins un moyen de renvoi (60).

9. Doigt artificiel selon la revendication 8,
**caractérisé en ce que**
le moyen de renvoi (60) est monté mobile en translation ou en basculement sur la phalange successive (30).

10. Doigt artificiel selon l'une des revendications 6 à 9,
**caractérisé en ce que**
le moyen de traction (50) est sollicité par une précontrainte en direction distale.

11. Doigt artificiel selon l'une des revendications précédentes,
**caractérisé en ce que**
l'entraînement (40) est réalisé sous forme d'entraînement linéaire autobloquant ou sous forme d'entraînement pneumatique non bloquant.

12. Doigt artificiel selon l'une des revendications précédentes,
**caractérisé en ce que**
l'entraînement (40) comprend un démultiplicateur (42) qui est couplé à une roulette ou à une transmission à broche (44) sur laquelle est fixé le moyen de traction (50).

13. Doigt artificiel selon l'une des revendications précédentes,
**caractérisé en ce que**
l'entraînement (40) est agencé dans la première phalange (20) ou dans la phalange successive (30).

14. Doigt artificiel selon l'une des revendications précédentes,
**caractérisé en ce que**
un point d'inversion (P) d'une attaque de force entre la flexion et l'extension est agencé entre la phalange (20) extensible et/ou la phalange successive (30), et le point d'attaque de force est réalisé mobile.
